# EUROPEAN PATENT SPECIFICATION

(11) **EP 2 345 435 B1**
(45) Date of publication and mention of the grant of the patent: **21.09.2016**
(21) Application number: 09820727.7
(22) Date of filing: 12.10.2009
(51) Int. Cl.: A61L 27/36, A61L 27/56, A61L 27/38

(54) **METHOD FOR MANUFACTURING A POROUS THREE-DIMENSIONAL SUPPORT USING POWDER FROM ANIMAL TISSUE, AND POROUS THREE-DIMENSIONAL SUPPORT MANUFACTURED BY SAME**
VERFAHREN ZUR HERSTELLUNG EINES PORÖSEN DREIDIMENSIONALEN SUBSTRATS ANHAND VON PULVER AUS TIERGEWEBE UND DAMIT HERGESTELLTES PORÖSES DREIDIMENSIONALES SUBSTRAT
PROCÉDÉ POUR LA FABRICATION D'UN SUPPORT POREUX TRIDIMENSIONNEL UTILISANT UNE POUDRE DÉRIVÉE DE TISSU ANIMAL, ET SUPPORT POREUX TRIDIMENSIONNEL FABRIQUÉ PAR CE PROCÉDÉ

(30) Priority: 13.10.2008 KR 20080100005
(43) Date of publication of application: 20.07.2011
(73) Proprietor: Ajou University Industry-Academic Cooperation Foundation, Suwon-si, Gyeonggi-do 443-749 (KR)
(72) Inventor: MIN, Byoung-Hyun, Anyang-si Kyonggi-do 431-076 (KR); JANG, Ji Wook, Suwon-si Kyonggi-do 443-380 (KR)
(74) Representative: Isarpatent
(86) International application number: PCT/KR2009/005839
(87) International publication number: WO 2010/044577

(56) References cited:
- WO-A2-2006/099332
- US-A- 4 656 137
- US-A1- 2007 248 638
- US-A1- 2008 124 374
- US-B2- 7 201 917
- YANG ET AL: "A cartilage ECM-derived 3-D porous acellular matrix scaffold for in vivo cartilage tissue engineering with PKH26-labeled chondrogenic bone marrow-derived mesenchymal stem cells", BIOMATERIALS, ELSEVIER SCIENCE PUBLISHERS BV., BARKING, GB, vol. 29, no. 15, 4 March 2008 (2008-03-04) , pages 2378-2387, XP022526910, ISSN: 0142-9612, DOI: 10.1016/J.BIOMATERIALS.2008.01.037
- Peter X Ma: "Scaffolds for tissue fabrication", Materials Today, 1 May 2004 (2004-05-01), pages 30-40, XP055085102, Retrieved from the Internet: URL:http://ac.els-cdn.com/S136970210400233 0/1-s2.0-S1369702104002330-main.pdf?_tid=6 b46d700-3bec-11e3-b67e-00000aab0f27&acdnat =1382537415_3b19c3c65ff3b6fbc164bf9a07f6b5 5a [retrieved on 2013-10-23]

## Description

### [Technical Field]

The present invention relates to a method for manufacturing a porous three-dimensional scaffold using powder from animal-derived tissue, more particularly to a method for manufacturing a porous three-dimensional scaffold, in which powder from animal-derived tissue is manufactured into a porous scaffold in a three-dimensional structure using a particle leaching method so as to construct a porous three-dimensional scaffold that may have various size, porosity, shape and structure, depending upon its purpose for treatment or use.

### [Background Art]

Articular chondrocytes are specialized mesoderm-derived cells found exclusively in cartilage. Cartilage is an avascular tissue composed of extracellular matrix produced by chondrocytes. It neither causes an inflammation reaction, nor induces a self-regeneration, when being damaged. Once cartilage is damaged, its self-regeneration is extremely limited to ultimately cause osteoarthritis, which largely affects the quality of patients' lives.

Presently, typical methods for treating a damaged cartilage may include bone marrow stimulation such as abrasion arthroplasty and microfracture, etc., mosaicplasty technique characterized by transplanting an osteochondral tissue to damaged areas, autologous chondrocyte implantation (ACI) characterized by culturing and transplanting autologous chondrocytes to damaged areas, and the like.

The bone marrow stimulation has been widely used since it requires a minimal invasion operated under an arthroscope for a short period of time. This method is advantageous due to its simple operation procedure and short operation time, but has a critical limation being unable to maintain blood clots (including stem cells) well that are essential to cartilage repair. Furthermore, the blood clots formed in this technique are physically unstable and not likely to regenerate efficiently a normal cartilage. Particularly, the loss of blood clots mostly causes that the regenerated cartilage is likely to become a fibrous cartilage rather than a normal hyaline cartilage. Hence, it is difficult to expect a successful healing of cartilage defect by using this bone marrow stimulation techniques.

In addition, the mosaicplasty technique is to fill damaged cartilage area with normal osteochondral tissues isolated from the areas with less weight bearing and low friction. This technique is outstanding to treat the damaged cartilages, but problematic to provoke a secondary damage during the isolation of osteochondral tissues.

Recently, the autologous chondrocyte implantation (ACI) characterized by culturing and transplanting autologous chondrocytes to damaged areas is used to treat a damaged cartilage. The implantation ACI is a clinically approved protocol to treat cartilage damage (Brittberg, M. et al., New Eng. J. Med., 331:889, 1994). However, it also has a problem that the area of cartilage damage should be covered with periosteum and tightly sutured after injecting chondrocytes. Moreover, the periosteum may allow chondrocytes to overgrow, which may cause pain at damaged areas after the surgery. The ACI is also disadvantageous to undergo two steps of surgery processes; isolating chondrocytes under an arthroscopic operation and culturing them for a long time *in vitro*, and then transplanting a cell suspension into damaged areas.

The present inventors have conceived that a three-dimensional scaffold could provide enhanced effects capable of regenerating a hyaline cartilage tissue when implanted in the cartilage defect. The three-dimensional scaffold is expected to have no limitation of its physical property, porosity, shape, structure and size if it is manufactured by using "a cartilage powdering process" and "a particle leaching method" after isolating animal tissues, especially animal cartilages.

Recently, it is noted that allogenic or xenogenic tissues or organs are directly harvested from an animal, then acellularized and applied for various types of scaffold or membrane.

Until now, small intestine submucosa (SIS), urinary bladder submucosa (UBM), skin, human amniotic membrane (HAM) and the like are already commercialized or being developed. For example, "Alloderm" was developed by decellularizing skin tissue from a donor, "OASIS" was developed as a dressing agent for wounds by decellularizing small intestine submucosa (SIS), and urinary bladder submucosa (UBM) was developed by decellularizing porcine uninary bladder tissue. In addition, "Chondro-gide", a bilayer membrane made of type I and type III collagens, was developed and under intensive research to regenerate a cartilage defect.

In detail, the cartilage tissue comprises tissue fluid, macromolecules and other substrates in view of its biochemical composition. The tissue fluid is composed mainly of water (65∼80%), and further includes proteins, inorganic salts, gas and various metabolites. The tissue fluid generally has positive charges at a high concentration to counter-balance the negative-charged proteoglycans.

About 60% of the macromolecules are composed of collagens. Collagens bind with proteoglycans in extracellular matrix to form a network structure of macromolecules, which maintains the integiry of cartilage structure and confer tension and elasticity on cartilage tissue. Most of cartilage collagens, i.e., 90∼95%, is comprised of type II collagen, in which the type II collagen assembles three strands of α-type chain into a helical structure to form a fibrillar shape. The proteoglycan is a complex molecule that has core proteins bound with glycosaminoglycans (GAG) including chondroitin 4-sulfate, chondroitin 6-sulfate, keratan sulfate and the like. Most of the proteoglycans, i.e., 80∼90% aggregate in cartilage to form a large molecule referred to as aggrecan.

The other substrates including non-collagenous proteins, glycoproteins and the like constitute 10∼15% of cartilage in dry weight. These substrates mainly play roles to stabilize the structure of macromolecules and help to form an organic tissue.

Unfortunately, a certain cellular antigen may cause an inflammation reaction or an immune rejection by being recognized by a host, when implanting a xenogenic and allogenic tissue to the host. Nevertheless, the components of extracellular matrix are generally resistant to those adverse responses of an allogenic recipient. Therefore, various extracellular matrices present in tissues including cardiovascular system, blood vessel, skin, nerve, skeletal muscle, tendon, urinary bladder, liver and the like have been actively investigated for applying to tissue-engineering and regenerative medicine. The purpose of decellularization from extracellular matrix is to reduce the rejection responses against the cellular components of the extracellular matrix and to increase the mechanical strength of the extracellular matrix by effectively removing cells, nuclei, etc.

The optimized and commonly used decellularization method includes physical and chemical methods. The physical method comprises stirring, ultrasonication, mechanical pressing, and freezing/thawing process. The physical method can destruct cell membranes and expose cellular components. After that, washing process should be conducted to remove cells from the extracellular matrix. But, the physical method is usually considered as insufficient for complete decellularization. Therefore, the physical method should be performed in combination with chemical methods. Enzymatic digestion using trypsin, or chemical treatment using an ionic solution can destruct cell membranes and break the connection between the inner side and the outer side of cells. During the decellularization, the extracellular matrix is properly disintegrated while maintaining its basal backbone. The decellularization can remove cellular materials and debris from tissue and expose all cells adequately to a chaotropic solution. The purpose of the decellularization process is mostly to maintain intact mechanical property or biological property of tissue with minimizing its destruction.

With regard to the above-mentioned purpose, conventional techniques for manufacturing a porous scaffold by using extracellular matrix from natural tissue have been disclosed. In particular, U.S. Patent Publication No. 2007/0248638A1 described that natural tissue cells such as chondrocytes may be decellularized by treating oxidant and detergent simultaneously and freeze-dried to form a porous scaffold. U.S. Patent Publication No. 2008/0124374A1 disclosed a method for decellularizing extracellular matrix in bone marrow cells from a vertebrate animal and a therapeutic device using the same.

The above-mentioned prior arts relate to the technique that a porous three-dimensional scaffold is formed from a xenogenic extracellular matrix obtained by decellularizing tissues made of natural chondrocytes or bone marrow cells. The prior arts could reduce possible immune rejection since a porous three-dimensional scaffold is manufactured by decellularizing a natural tissue, however, they have a problem that there are limitations concerning the size, porosity, shape and structure of the scaffold due to using a natural tissue itself. Accordingly, it was difficult to apply the prior arts to commercial purpose and therapeutic use.

That is to say, it is necessary to vary the size, shape or structure of a porous three-dimensional scaffold used for treatment, depending upon damaged site, damaged area and characteristics of a patient. But, the prior arts of simply decellularizing natural cartilage tissue could not meet the aforesaid needs because of obstructing diversification for treatment.

Meanwhile, U.S. Patent No. 7,201,917 disclosed a method for manufacturing a porous scaffold by forming extracellular matrix in a liquid into a slurry and then freeze-drying the slurry, but only directed to obtaining a liquid-phase slurry from extracellular matrix of natural tissue. In addition, U.S. Patent No. 4,656,137 disclosed a method for manufacturing an animal cartilage powder, comprising collecting cartilage from an animal; removing various proteins and lipid tissues attached on the cartilage by treating enzymatic agents; pulverizing primarily to 4∼8 mm of size; removing moisture through lyophilization; and reducing to powder in 40∼70 µm of size.

WO 2006/099332 discloses a scaffold for reconstructing cartilage or bone structures, wherein the chondrocyte seeded scaffold comprises a porous structure obtained from decellularized tissue such as submucosa by salt leaching. EDC/HNS is used to crosslink and immobilize compounds to the scaffold. The submucosa was decellularized and powdered before forming it into a porous structure. WO 2006/099332 discloses cross-linking of porous structures, however, not for the particular embodiment employing not dissolved animal-derived and powdered material.However, the above-mentioned methods have a problem that there are limitations concerning the size, porosity, shape and structure of the scaffold due to using extracellular matrix obtained by decellularizing a natural tissue itself. Accordingly, it was difficult to apply the above-mentioned methods to commercial purpose and therapeutic use. U.S. Patent No. 4,656,137 merely disclosed a method of reducing to cartilage powder for treating wounds, but ignored a three-dimensional scaffold prepared from cartilage powder having various sizes, shapes and structures.

Accordingly, it needs in the art to develop a technique for providing a three-dimensional scaffold with enhanced effects capable of regenerating a hyaline cartilage tissue without any limitation of its physical property, porosity, shape, structure and size.

In order to solve the aforesaid problems of the conventional methods, we have developed a porous three-dimensional scaffold without any limitation of its physical property, porosity, shape, structure and size by using "a cartilage powdering process" and "a particle leaching method" after isolating an animal tissue, for example, an animal cartilage.

The particle leaching method is utilized in the tissue-engineering field to manufactured a three-dimensional scaffold from synthetic polymers including PLA, PGA, PLGA, etc. In the particle leaching method, a three-dimensional scaffold having a desired pore size can be manufactured by the following steps: adding a porogen having a desired pore size (salts, organic sugars, paraffin, ice particles and the like) into a certain amount of polymer solution; mixing them; molding them to a desired shape; casting or lyophilizing them to remove organic solvents completely; and dissolving the porogen with water, proper solvents, etc., or removing the porogen by drying it.

The present inventors have adopted the basic principle of the aforesaid particle leaching method, but mixed animal-derived tissue powder with a porogen without dissolving them in an organic solvent or any other solvent, and used a cross-linking agent (e.g., EDC) to aggregate the powder.

Furthermore, it is confirmed that a porous three-dimensional scaffold made of animal tissue powder, for example, cartilage powder is remarkably biocompatible and clinically applicable without any inflammation when being implanted if the porous three-dimensional scaffold is manufactured by decellularizing the animal tissue before or after powdering it, or simultaneously with powdering it.

### [Disclosure]

### [Object]

The object of the present invention is to provide a method for manufacturing a porous three-dimensional scaffold, in which powder from an animal tissue, for example, an animal cartilage is manufactured into a porous scaffold in a three-dimensional structure using a particle leaching method so as to construct a porous three-dimensional scaffold that may have various size, porosity, shape and structure, depending upon its purpose for treatment or use.

More particularly, the object of the present invention is to provide a method for manufacturing a porous three-dimensional scaffold with enhanced effects capable of regenerating a hyaline cartilage tissue without any limitation of its physical property, porosity, shape, structure and size using "a cartilage powdering process" and "a particle leaching method" after isolating an animal tissue, for example, an animal cartilage.

Further, the object of the present invention is to provide a method for manufacturing a porous three-dimensional scaffold, in which a porous three-dimensional scaffold is remarkably biocompatible and clinically applicable without any inflammation when being implanted if the porous three-dimensional scaffold is manufactured by physically and/or chemically decellularizing an animal tissue before or after powdering it, or simultaneously with powdering it after isolating an animal tissue, for example, an animal cartilage.

### [Technical Solution]

In order to accomplish the above-mentioned objects, a method of the present invention for manufacturing a porous three-dimensional scaffold using an animal tissue powder comprises the following steps of: (a) powdering an animal-derived tissue; (b) decellularizing the animal-derived tissue before or after powdering it, or simultaneously with powdering it; (c) mixing the decellularized animal-derived tissue powder with a porogen without dissolving them in an organic solvent or any other solvent, and adding a cross-linking agent to the mixture so as to crosslink and aggregate the decellularized animal-derived tissue powder; (d) after step (c), forming the crosslinked and decellularized animal-derived tissue powder into a three-dimensional scaffold; and (e) forming a porous three-dimensional scaffold by treating the three-dimensional scaffold with a particle leaching method which uses as the porogen at least one particle selected from a group consisting of a salt particle such as sodium chloride, an organic sugar particle, a dextran particle, a sucrose particle, an ice particle and a paraffin particle such that the porogen is removed from the three-dimensional scaffold.

In a preferred embodiment of the method of the present invention the decellularized animal-derived tissue powder is formed into a porous three-dimensional scaffold by mixing the decellularized animal-derived tissue powder and porogen particles used in the particle leaching method, adding the cross-linking agent to the mixture, pouring them into a mold, and then molding them under pressure to construct a porous three-dimensional scaffold having various shapes and sizes depending upon its purpose for use and its application site for treatment.

In a preferred embodiment the method of the present invention comprises a further cross-linking of the three-dimensional scaffold obtained from step (d) by at least one selected from a group consisting of UV, EDC, NBS, dehydrothermal method and glutaraldehyde.

In a preferred embodiment the method of the present invention further comprises adding at least one growth factor to the animal-derived tissue powder and freeze-drying them.

In a preferred embodiment the method of the present invention further comprises inoculating chondrocytes on or in the porous three-dimensional scaffold formed by the particle leaching method, then re-culturing the chondrocytes on or in the porous three-dimensional scaffold and obtaining a tissue-engineered cartilage tissue.

In a preferred embodiment of the method of the present invention the animal-derived tissue may be cartilage, or an amniotic membrane derived from pigs, cattle, sheep, horses, dogs or cats.

In a preferred embodiment of the method of the present invention the step of powdering the animal-derived tissue comprises isolating cartilage from an animal-derived cartilage tissue, pulverizing the cartilage by a pulverizer, and reducing the pulverized cartilage to powder by a freezing mill.

In a preferred embodiment of the method of the present invention the decellularization is performed by physical decellularization, chemical decellularization, or the combination of the physical decellularization and the chemical decellularization.

In a preferred embodiment of the method of the present invention the physical decellularization includes freezing-thawing, ultrasonication, or physical agitation, and the chemical decellularization is performed by treating the animal-derived tissue powder with hypotonic solution, anionic detergent, non-ionic detergent, cationic detergent, DNase, RNase or trypsin.

In a preferred embodiment of the method of the present invention in the chemical decellularization, the hypotonic solution is Tris HCl (pH 8.0) solution, the anionic detergent is sodium dodecyl sulfate (SDS), sodium deoxycholate, or Triton X-200, the non-ionic detergent is Triton X-100, and the cationic detergent is CHAPS, Sulfobetaine-10 (SB-10), Sulfobetaine-16 (SB-16), or tri-n-butyl phosphate.

### [Advantageous Effects]

The porous three-dimensional scaffold of the present invention manufactured by an animal tissue, for example, an animal cartilage powder can be advantageously applied in clinical fields, depending upon its purpose for treatment or use since it has various size, porosity, shape and structure. The present invention can provide the porous three-dimensional scaffold with enhanced effects capable of regenerating a hyaline cartilage tissue without any limitation of its physical property, porosity, shape, structure and size.

In addition, the porous three-dimensional scaffold of the present invention manufactured by an animal tissue, for example, an animal cartilage powder is remarkably biocompatible and clinically applicable without any immune rejection or inflammation when being implanted since the porous three-dimensional scaffold is manufactured by physically and/or chemically decellularizing an animal tissue before or after powdering it, or simultaneously with powdering it after isolating an animal tissue, for example, an animal cartilage. Furthermore, it is remarkably effective upon regenerating cartilage, as compared with scaffolds composed of collagen and other synthetic polymers.

Besides, the porous three-dimensional scaffold of the present invention manufactured by an animal tissue, for example, an animal cartilage powder can provide an environment suitable for cell migration, cell growth, and cell differentiation because it is manufactured to have a three-dimensional structure. Furthermore, the porous three-dimensional scaffold can be comprised of growth factors and proper components suitable for regenerating cartilage. The porous three-dimensional scaffold of the present invention can be usefully applied for a tissue-engineered scaffold of treating cartilage loss, due to its outstanding bio-compatibility, bio-degradable ability and three-dimensional structure.

### [Brief Description of Drawings]

The above and other objects, features and other advantages of the present invention will be more clearly understood to those skilled in this arts from the following detailed description taken in conjunction with the accompanying drawings.
FIG. 1(a) is a photograph of a porcine cartilage fragment separated from porcine cartilage, FIG. 1(b) is a photograph of the porcine cartilage powder that was decellularized after being freezing-pulverized, and FIG. 1(c) is a SEM photograph of the decellularized porcine cartilage powder.
FIG. 2 is a graph that shows the DNA contents remained in natural porcine cartilage (PC before decellularization), decellularized porcine cartilage fragment (decellularized PC), and decellularized porcine cartilage powder (decellularized PCP).
FIG. 3 is a schematic diagram that shows the process for manufacturing the porous three-dimensional scaffold using cartilage powder according to the present invention.
FIG. 4 is a photograph of the porous three-dimensional scaffold manufactured by cartilage powder according to one embodiment of the present invention.
FIG. 5 is a SEM photograph of the porous three-dimensional scaffold manufactured by cartilage powder according to one embodiment of the present invention. FIG. 5(a) is a 20 x -magnified photograph of the surface, FIG. 5(b) is a 50 x -magnified photograph of the surface, FIG. 5(c) is a 20 x -magnified photograph of the side, and FIG. 5(d) is a 50 x -magnified photograph of the side.
FIG. 6 is a distributional graph showing porosity in the porous three-dimensional scaffold manufactured by cartilage powder according to one embodiment of the present invention.
FIG. 7 is a photograph that shows the experimental data of hydrophilic property in the porous three-dimensional scaffold manufactured by cartilage powder according to one embodiment of the present invention
FIG. 8 represents photographs of the collagen sponge and the porcine cartilage powder scaffold of the present invention, taken before evaluating their efficacies by culturing cells under *in vitro* condition. FIG. 8(a) is a photograph of the front view of the collagen sponge, FIG. 8(b) is a photograph of the side view of the collagen sponge, FIG. 8(c) is a photograph of the front view of the porcine cartilage powder scaffold of the present invention, and FIG. 8(d) is a photograph of the side view of the porcine cartilage powder scaffold of the present invention.
FIG. 9 is a table showing the result of analyzing cell inoculation rates after inoculating cells on or in the collagen sponge as a control group and the porcine cartilage powder scaffold of the present invention.
FIG. 10 represents photographs showing the growth of the collagen sponge as a control group and the porcine cartilage powder scaffold of the present invention, in a week after *in vitro* culturing. FIG. 10(a) is a photograph of the front view of the collagen sponge, FIG. 10(b) is a photograph of the side view of the collagen sponge, FIG. 10(c) is a photograph of the front view of the porcine cartilage powder scaffold of the present invention, and FIG. 10(d) is a photograph of the side view of the porcine cartilage powder scaffold of the present invention.
FIG. 11 represents Safranin-O staining photographs of the collagen sponge as a control group and the porcine cartilage powder scaffold of the present invention, in a week after *in vitro* culturing. FIG. 11(a) is a 20 x -magnified photograph of the collagen sponge, FIG. 11(b) is a 100 x -magnified photograph of the collagen sponge, FIG. 11(c) is a 20 x - magnified photograph of the porcine cartilage powder scaffold of the present invention, and FIG. 11(d) is a 100 x -magnified photograph of the porcine cartilage powder scaffold of the present invention.
FIG. 12 represents photographs showing the growth of the collagen sponge as a control group and the porcine cartilage powder scaffold of the present invention, in 2 weeks after *in vitro* culturing. FIG. 12(a) is a photograph of the front view of the collagen sponge, FIG. 12(b) is a photograph of the side view of the collagen sponge, FIG. 12(c) is a photograph of the front view of the porcine cartilage powder scaffold of the present invention, and FIG. 12(d) is a photograph of the side view of the porcine cartilage powder scaffold of the present invention.
FIG. 13 represents Safranin-O staining photographs of the collagen sponge as a control group and the porcine cartilage powder scaffold of the present invention, in 2 weeks after *in vitro* culturing. FIG. 13(a) is a 20 x -magnified photograph of the collagen sponge, FIG. 13(b) is a 100 x -magnified photograph of the collagen sponge, FIG. 13(c) is a 20 x -magnified photograph of the porcine cartilage powder scaffold of the present invention, and FIG. 13(d) is a 100 x -magnified photograph of the porcine cartilage powder scaffold of the present invention.

### [Mode for Invention]

Practical and presently preferred embodiments of the present invention are illustrated more clearly as shown in the following examples.

### Examples

### Reference Example 1: Isolation of porcine cartilage

In order to isolate porcine cartilage, pig cartilage was purchased and utilized from a facility satisfying standards referred to EN 12442 "Animal tissues and their derivatives utilized in the manufacture of medical devices, Part 1; Analysis and management of risk, Part 2; Controls on sourcing, collection and handling".

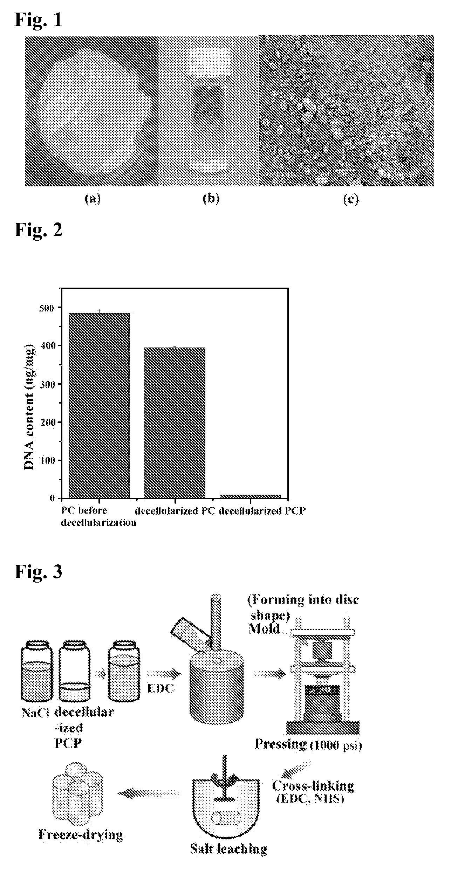

Cartilage tissue was isolated from the porcine cartilage and cut into pieces (about 20 x 30 mm) and then, washed 3 times for 10 minutes by a saline solution. The resulting cartilage fragment was immersed in PBS solution containing antibiotic-antimycotic agents and finally stored at -80°C in an ultralow-temperature refrigerator (See FIG. 1(a)).

### Example 1: Pulverizing porcine cartilage

The cartilage fragment washed out was pulverized by a pulverizer that is commercially available and well-known to those skilled in this arts (Hood Mixer HMF-505, Hanil Co. Ltd. Korea) to reduce its size to about 2 x 2 mm. The pulverized cartilage fragment was freeze-dried and freeze-dried cartilage fragment was finally reduced to powder having its size of about 10µm by a freezing mill (JAI, JFC-300, Japan).

### 1-1. Morphological analysis of porcine cartilage powder

The resulting porcine cartilage powder was morphologically analyzed under a scanning electron microscope. The porcine cartilage powder prepared in Example 1 was fixed by 2.5% glutaraldehyde for about 1 hour, and washed by phosphate buffer solution. The sample was dehydrated, dried and observed under a microscope (JEOL, JSM-6380, Japan; 20KV) to measure the size and shape of the powder. The powder was observed in the size of about 10µm (FIG. 1(c)).

### Example 2: Decellularization and characterization of porcine cartilage power

### 2-1. Decellularization of porcine cartilage power

In order to remove chondrocytes and genetic components and obtain pure extracellular matrix, decellularization process was performed as below.

The porcine cartilage powder prepared in Example 1 was added to 1ℓ of 0.1% SDS (sodium dodecyl sulfate, Bio-Rad, USA)(per 10 g of the porcine cartilage powder) and stirred at 100 rpm for 24 hours. After treating SDS, the resultant was washed 5 times by tertiary distilled water at 100 rpm for 30 minutes.

In order to precipitate the cartilage powder for exchanging washing solution, the cartilage powder was centrifuged at 10,000 rpm for 1 hour with an ultracentrifuge (US-21 SMT, Vision, Korea).

200 ml of 200 U/ml DNase (Sigma, USA) was added to the cartilage powder and stirred at 100 rpm at 37°C for 24 hours. The resultant was washed 5 times by tertiary distilled water at 100 rpm for 30 minutes. The washing solution was exchanged under the same condition of centrifugation as conducted in the above-mentioned SDS washing. The decellularized cartilage powder is shown in FIG. 1(b).

In this example of the present invention, the decellularization was performed after preparing cartilage powder, but it is clearly understood to those skilled in the arts that the present invention does not exclude the process for decellularizing cartilage and powdering it before reducing it to cartilage powder.

### 2-2. Analysis of DNA content of decellularized porcine cartilage powder

Intact cartilage tissue before decellularization (hereinafter, referred to as "PC"), decellularized cartilage fragment (PC) and cartilage powder after decellularization (hereinafter, referred to as "PCP") were prepared for samples and their DNA contents were measured quantitatively with a Qubic DNA quantitation device (Qubic, Bio-Rad, USA). The result from the quantitative analysis of DNA contents is illustrated in Table 1 and FIG. 2 as below. Particularly, the decellularization was performed according to the same procedure as described in Example 2-1.

**Table 1**

| **Measurement of residual amounts of DNAs** | | |
|---|---|---|
| | DNA contents (residual amounts) | C.V values |
| PC before decellularization | 485 | 13.22876 |
| PC after decellularization | 394 | 4.358899 |
| PCP after decellularization | 9.48 | 0 |

### Example 3: Manufacturing and characterizing three-dimensional scaffold using decellularized porcine cartilage powder

### 3-1. Manufacturing porous three-dimensional scaffold

Decellularized cartilage powder prepared by the same procedure as described in Example 2-1 was uniformly mixed with sodium chloride (having crystal size of 250 to 350 µm) in the ratio of 1:9. 100 mM EDC (N-(3-dimethylaminopropyl)-N-ethylcarbodiimide hydrochloride)(Sigma, USA) solution in tertiary distilled water was added to the mixture in the ratio of 10%.

The EDC solution was uniformly blended with the mixture of the cartilage powder and the sodium chloride. The resulting sample of the mixture was poured into a self-designed mold made of cemented carbides and molded under 1000 psi of pressure to obtain a disc-shaped product. The mold is composed of a fixing member and a moving member and forms a desired three-dimensional scaffold in the inside of the fixing member and/or the moving member. In this example, the disc-shaped three-dimensional scaffold was manufactured, but another three-dimensional scaffolds having various shapes and sizes can be manufactured, depending upon their purpose for use and treatment.

In this example, sodium chloride was utilized as a porogen in order to manufacture a porous three-dimensional scaffold, but it is clearly understood to those skilled in the arts that other substances including sugar particles or ice particles can be utilized in the method of the present invention.

After that, the disc-shaped product was dried at room temperature and then crosslinked by 100 mM EDC solution (dissolved in 99.9% ethanol) for 4 hours. 100 mM NHS (N-hydroxysuccinimide, Fluka, Japan) was added to the reactant and reacted for 4 hours again. The cross-linking reaction was performed to improve the physical property and elongate the degradation period in the porous three-dimensional scaffold made of cartilage powder.

After the cross-linking reaction, the resulting disc-shaped product was immersed in tertiary distilled water and the tertiary distilled water was exchanged at least 5 times to completely elute salts out from the resulting disc-shaped product. In order to remove nonreactive groups after cross-linking, the resulting disc-shaped product was washed 3 times by NH₂PO₄. Finally, it was washed 3 times again by tertiary distilled water and freeze-dried.

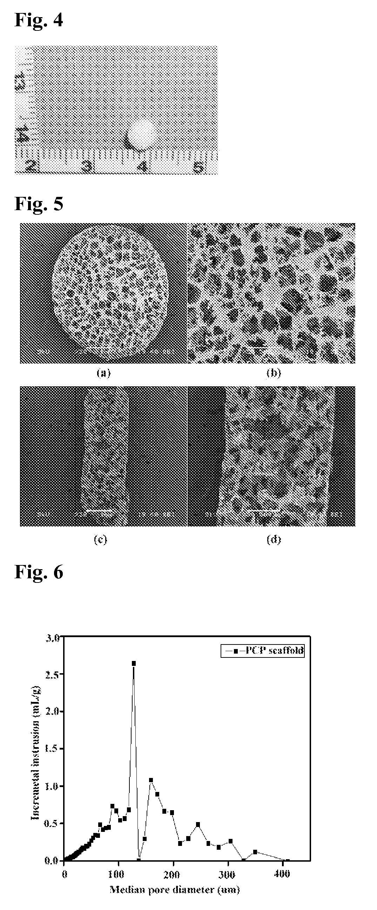

FIG. 3 shows the process for manufacturing the porous three-dimensional scaffold using a cartilage powder, and FIG. 4 shows a final product manufactured by the process.

### 3-2. Structural analysis of pore structure of porcine cartilage powder scaffold

The pore structure of porcine cartilage powder scaffold was analyzed under a scanning electron microscope. The porcine cartilage powder scaffold prepared in Example 3-1 was fixed by 2.5% glutaraldehyde for about 1 hour, and washed out by phosphate buffer solution. The resulting sample was dehydrated with ethanol, dried and then analyzed under a microscope (JEOL, JSM-6380, Japan; 20 KV) to observe the pore size and shape of the porcine cartilage powder scaffold.

The pores of scaffold were observed in the size of about 200 µm and these pores were well inter-connected with each other. Further, the pores were uniformly distributed and the surface pores of the scaffold remained open (See FIG. 5).

### 3-3. Analysis of porosity of porcine cartilage powder scaffold

The porosity of porcine cartilage powder scaffold was analyzed with a mercury porosimeter. The analyzed sample weight was measured to 0.0193 g. The conditions for analysis were as follows:
- pressure: 50 µmHg;
- time: 5 minutes; and
- mercury-filling pressure 0.44 psia.

As a result, the porosity of the porcine cartilage powder scaffold was measured to 82.39% (See FIG. 6).

### Example 4: Evaluation of hydrophilic property of porcine cartilage powder scaffold

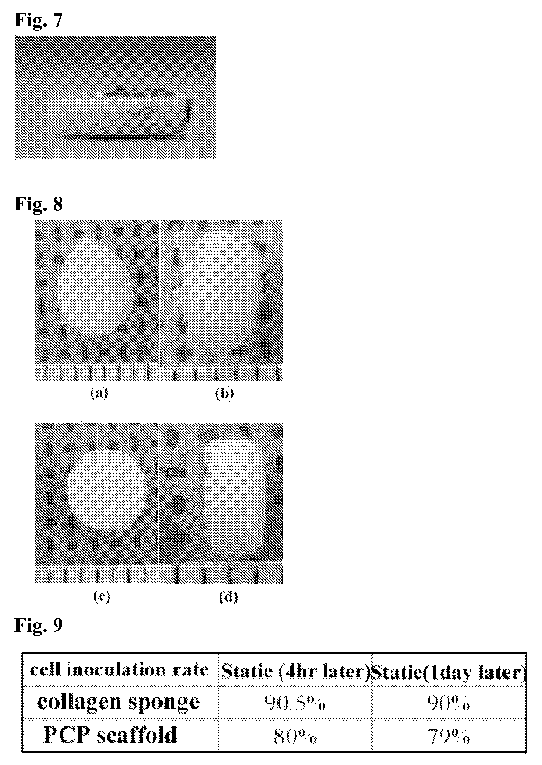

In order to examine the hydrophilic property of the porcine cartilage powder scaffold, its ability of absorbing water was observed using a dye with naked eyes. 2.5% Trypan blue of cell-staining solution was dropped to the porcine cartilage powder scaffold. A photograph was taken 10 seconds later and it was observed by the photograph for the porcine cartilage powder scaffold to absorb the dye immediately. Therefore, it was confirmed that the porcine cartilage powder scaffold of the present invention shows remarkable hydrophilic property (See FIG. 7).

### Example 5: Evaluation of efficacy of porcine cartilage powder scaffold

### 5-1. Culturing of chondrocytes on or in porcine cartilage powder scaffold

Chondrocytes were separated from cartilage of ∼2 weeks old New Zealand white rabbits. Cartilage tissues were exclusively isolated from the articular cartilage of the knee joint, then cut finely into pieces of about 1∼2 mm, and treated with 0.1% collagenase (type II, Washington, USA) in a cell incubator at 37°C for 12 hours to isolate chondrocytes. After treating 0.1% collagenase for 12 hours, chondrocytes were selected by a cell filtrator and centrifuged (1,700 rpm, 10 minutes) to isolate chondrocytes exclusively.

The chondrocytes isolated from rabbits were seeded at the concentration of 5 x 10⁶ cells on or in the porous three-dimensional cartilage powder scaffold (5 mm of radius, 2 mm of height) prepared by the procedures described in Examples 1∼3.

Then, cell culture media [DMEM + 1% antibiotic-antimycotic + ITS comprising "1.0 mg/ml of insulin, 0.55 mg/ml of human transferrin and 0.5 mg/ml of sodium selenite" + 50 µg/ml of ascorbic acid + 1.25 mg/ml of bovine serum albumin + 100 nM dexamethasone + 40 µg/ml of proline] were freshly exchanged 3 times a week.

### 5-2 Evaluation of cell inoculation rate on or in porcine cartilage powder scaffold

Chondrocytes of rabbits were counted to 5 x 10⁶ cells and inoculated on or in the porcine cartilage powder scaffold by static seeding. The inoculated cells were incubated at 37°C for 4 hours in a cell incubator to allow the cells to attach on or in the scaffold, and then culture media were poured into the scaffold. At that time, free cells that were not attached on or in the scaffold and dropped out of it were counted. The scaffold was transferred to a fresh plate 24 hours later and free cells dropped on the bottom of the plate were counted again.

As a result, it was observed that the cell inoculation rate of the collagen sponge and the porcine cartilage powder scaffold after 4 hours was measured to 90.5% and 80%, respectively. Further, it was observed that the cell inoculation rate of the collagen sponge and the porcine cartilage powder scaffold after 24 hours was measured to 90% and 79% , respectively.

Even though the collagen sponge showed a higher cell inoculation rate than the porcine cartilage powder scaffold, it was confirmed that the cartilage powder scaffold also showed a high cell inoculation rate of about 80% (See FIG. 9).

### 5-3. Comparison of cartilage tissue formation over time

Type I atellocollagen (MATRIXEN™, Bioland, Korea) was dissolved in the concentration of 1% and then made to a sponge by freeze-drying it. The resulting sponge was used as a control group compared with the scaffold of the present invention.

As described above, the collagen sponge for a control group and the cartilage powder scaffold of the present invention were cultured for 1 week and 2 weeks after being seeded by rabbit chondrocytes. Then, the samples were recovered after some period and fixed by 10% formalin for 24 hours. The fixed samples were imbedded in paraffin, cut into sections and stained with Safranin-O and H&E staining to compare the ability of forming cartilage tissue between the collagen sponge and the cartilage powder scaffold.

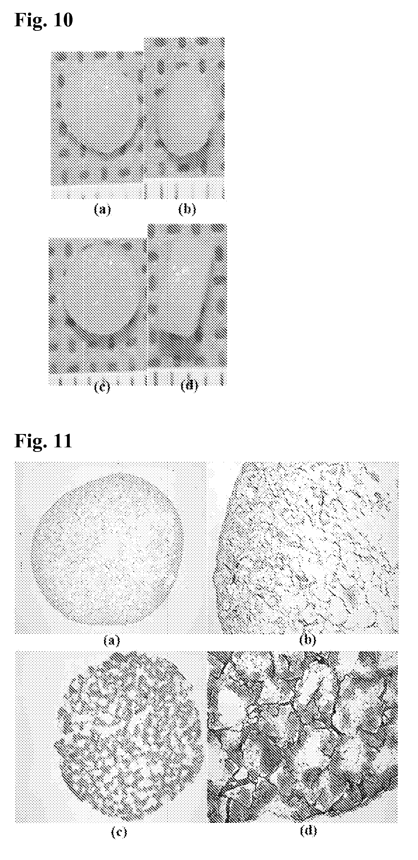

As a result, it was observed that white-colored semi-transparent tissues were formed in a week after culturing rabbit chondrocytes in the collagen sponge and the cartilage powder scaffold(See FIG. 10). When being stained with Safranin-O, chondrocytes were distributed both in the collagen sponge and in the cartilage powder scaffold. But, the cartilage powder scaffold of the present invention showed a higher cell distribution than the collagen sponge. Further, the cartilage powder scaffold of the present invention was superior to the collagen sponge in view of the synthesis of glycosaminoglycan and the progress of ossifluence along the wall of the scaffold (See FIG. 10).

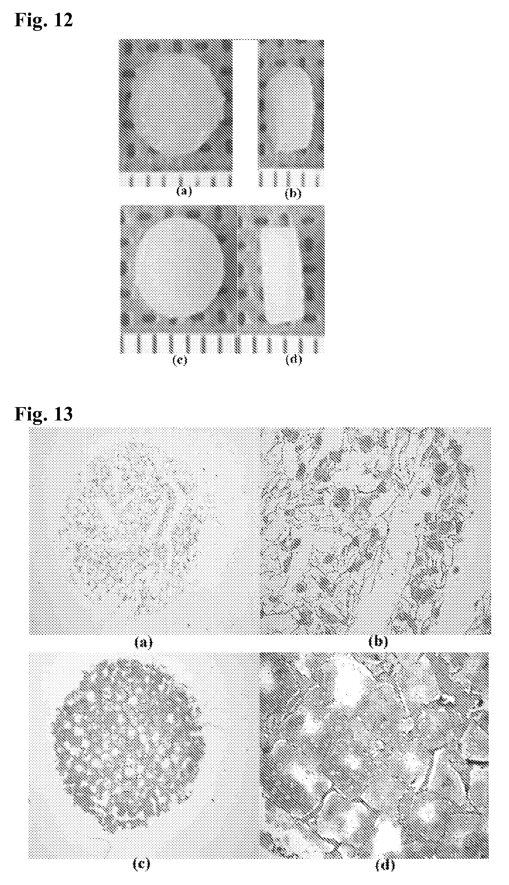

As shown in FIG. 11, both of two groups appeared semi-transparent in 2 weeks after culturing rabbit chondrocytes. It was observed by Safranin-O staining that both of two groups after 2 weeks were better than those after 1 week in view of the ability of regenerating cartilage. Especially, it was confirmed that the porous three-dimensional cartilage powder scaffold of the present invention can almost entirely regenerate cartilage (See FIG. 12).

Therefore, the porous three-dimensional scaffold of the present invention can regenerate cartilage without any limitation of its physical property, porosity, shape, structure and size, and provide an environment suitable for cell migration, cell growth, and cell differentiation because it is manufactured to have a three-dimensional structure. Furthermore, the porous three-dimensional scaffold of the present invention can be usefully applied for a tissue-engineered scaffold of treating cartilage loss, due to its outstanding bio-compatibility, bio-degradable ability and three-dimensional structure.

## Claims

1. A method for manufacturing a porous three-dimensional scaffold using animal tissue powder comprising:
(a) powdering an animal-derived tissue;
(b) decellularizing the animal-derived tissue before or after powdering it, or simultaneously with powdering it;
(c) mixing the decellularized animal-derived tissue powder with a porogen without dissolving them in an organic solvent or any other solvent, and adding a cross-linking agent to the mixture so as to crosslink and aggregate the decellularized animal-derived tissue powder;
(d) after step (c), forming the crosslinked and decellularized animal-derived tissue powder into a three-dimensional scaffold; and
(e) forming a porous three-dimensional scaffold by treating the three-dimensional scaffold with a particle leaching method which uses as the porogen at least one particle selected from a group consisting of a salt particle such as sodium chloride, an organic sugar particle, a dextran particle, a sucrose particle, an ice particle and a paraffin particle such that the porogen is removed from the three-dimensional scaffold.

2. The method as claimed in claim 1, wherein the decellularized animal-derived tissue powder is formed into a porous three-dimensional scaffold by mixing the decellularized animal-derived tissue powder and porogen particles used in the particle leaching method, adding the cross-linking agent to the mixture, pouring them into a mold, and then molding them under pressure to construct a porous three-dimensional scaffold having various shapes and sizes depending upon its purpose for use and its application site for treatment.

3. The method as claimed in any one of claim 1 or 2, further comprising a further cross-linking of the three-dimensional scaffold obtained from step (d) by at least one selected from a group consisting of UV, EDC, NBS, dehydrothermal method and glutaraldehyde.

4. The method as claimed in any one of claim 1 or 2, further comprising adding at least one growth factor to the animal-derived tissue powder and freeze-drying them.

5. The method as claimed in any one of claim 1 or 2, further comprising inoculating chondrocytes on or in the porous three-dimensional scaffold formed by the particle leaching method, then re-culturing the chondrocytes on or in the porous three-dimensional scaffold and obtaining a tissue-engineered cartilage tissue.

6. The method as claimed in any one of claim 1 or 2, wherein the animal-derived tissue may be cartilage, or an amniotic membrane derived from pigs, cattle, sheep, horses, dogs or cats.

7. The method as claimed in any one of claim 1 or 2, wherein the step of powdering the animal-derived tissue comprises isolating cartilage from an animal-derived cartilage tissue, pulverizing the cartilage by a pulverizer, and reducing the pulverized cartilage to powder by a freezing mill.

8. The method as claimed in any one of claim 1 or 2, wherein the decellularization is performed by physical decellularization, chemical decellularization, or the combination of the physical decellularization and the chemical decellularization.

9. The method as claimed in claim 8, wherein the physical decellularization includes freezing-thawing, ultrasonication, or physical agitation, and the chemical decellularization is performed by treating the animal-derived tissue powder with hypotonic solution, anionic detergent, non-ionic detergent, cationic detergent, DNase, RNase or trypsin.

10. The method as claimed in claim 9, wherein in the chemical decellularization, the hypotonic solution is Tris HCl (pH 8.0) solution, the anionic detergent is sodium dodecyl sulfate (SDS), sodium deoxycholate, or Triton X-200, the non-ionic detergent is Triton X-100, and the cationic detergent is CHAPS, Sulfobetaine-10 (SB-10), Sulfobetaine-16 (SB-16), or tri-n-butyl phosphate.

## Patentansprüche

1. Verfahren zum Herstellen eines porösen dreidimensionalen Gerüsts unter Verwendung von Tiergewebepulver, umfassend:
(a) Zerpulvern eines vom Tier abgeleiteten Gewebes;
(b) Dezellularisieren des vom Tier abgeleiteten Gewebes vor oder nach dem Zerpulvern desselben, oder gleichzeitig mit dem Zerpulvern desselben;
(c) Mischen des dezellularisierten vom Tier abgeleiteten Gewebepulvers mit einem Porogen, ohne sie in einem organischen Lösungsmittel oder jeglichem anderen Lösungsmittel zu lösen, und Zugeben eines Vernetzungsmittels zur Mischung, um das dezellularisierte vom Tier abgeleitete Gewebepulver zu vernetzen und zu aggregieren;
(d) Formen des vernetzten und dezellularisierten vom Tier abgeleiteten Gewebepulvers zu einem dreidimensionalen Gerüst nach Schritt (c); und
(e) Bilden eines porösen dreidimensionalen Gerüsts durch Behandeln des dreidimensionalen Gerüsts mit einem Partikel auswaschenden Verfahren, welches als Porogen zumindest ein Partikel verwendet, das ausgewählt ist aus der Gruppe, bestehend aus einem Salzpartikel wie Natriumchlorid, einem organischen Zuckerpartikel, einem Dextranpartikel, einem Saccharosepartikel, einem Eispartikel, und einem Paraffinpartikel, so dass das Porogen aus dem dreidimensionalen Gerüst entfernt wird.

2. Verfahren nach Anspruch 1, wobei das dezellularisierte vom Tier abgeleitete Gewebepulver zu einem dreidimensionalen Gerüst geformt wird, indem das dezellularisierte vom Tier abgeleitete Gewebepulver und Porogenpartikel, die in dem Partikel auswaschenden Verfahren verwendet werden, gemischt werden, das Vernetzungsmittel zu der Mischung zugegeben wird, diese in eine Form gegossen werden, und diese unter Druck geformt werden, um ein poröses dreidimensionales Gerüst zu bilden, das verschiedene Formen und Größen haben kann abhängig von dessen Verwendungszweck und dessen Anwendungsstelle zur Behandlung.

3. Verfahren nach einem der Ansprüche 1 und 2, weiter umfassend ein weiteres Vernetzen des in Schritt (d) erhaltenen dreidimensionalen Gerüsts durch zumindest einem, ausgewählt aus der Gruppe, bestehend aus UV, EDCI ("EDC"), NBS, einem dehydrothermalen Verfahren und Glutaraldehyd.

4. Verfahren nach einem der Ansprüche 1 und 2, weiter umfassend eine Zugabe von zumindest einem Wachstumsfaktor zum vom Tier abgeleiteten Gewebepulver und Gefriertrocknen derselben.

5. Verfahren nach einem der Ansprüche 1 und 2, weiter umfassend ein Einimpfen von Chondrozyten auf oder in das poröse dreidimensionale Gerüst, welches durch das Partikel auswaschende Verfahren gebildet wurde, dann Rekultivieren der Chondrozyten auf oder in dem porösen dreidimensionalen Gerüst und Erhalten eines gewebegezüchteten Knorpelgewebes.

6. Verfahren nach einem der Ansprüche 1 und 2, wobei das vom Tier abgeleitete Gewebe Knorpel, oder eine amniotische Membran, abgeleitet von Schweinen, Vieh, Schafen, Pferden, Hunden oder Katzen sein kann.

7. Verfahren nach einem der Ansprüche 1 und 2, wobei der Schritt des Zerpulverns des vom Tier abgeleiteten Gewebes ein Isolieren von Knorpel aus einem vom Tier abgeleiteten Knorpelgewebe, ein Zerpulvern des Knorpels durch eine Mühle, und ein Zerkleinern des zerpulverten Knorpels zu Pulver durch eine Gefriermühle umfasst.

8. Verfahren nach einem der Ansprüche 1 und 2, wobei das Dezellularisieren durch physikalisches Dezellularisieren, chemisches Dezellularisieren, oder der Kombination von physikalischem Dezellularisieren und chemischem Dezellularisieren durchgeführt wird.

9. Verfahren nach Anspruch 1, wobei das physikalische Dezellularisieren ein Gefrieren-Auftauen, Ultraschall, oder physikalisches Rühren bzw. Schütteln beinhaltet, und das chemische Dezellularisieren durch Behandeln des vom Tier abgeleiteten Gewebepulvers mit hypotonischer Lösung, anionischem Detergens, nicht-ionischem Detergens, kationischem Detergens, DNase, RNase, oder Trypsin durchgeführt wird.

10. Verfahren nach Anspruch 9, wobei beim chemischen Dezellularisieren die hypotonische Lösung eine Tris HCl (pH 8,0) Lösung ist, das anionische Detergens Natriumdodecylsulfat (SDS), Natriumdeoxycholat oder Triton X-200 ist, das nicht-ionische Detergens Triton X-100 ist, und das kationische Detergens CHAPS, Sulfobetain-10 (SB-10), Sulfobetain-16 (SB-16) oder Tri-n-butyl-phosphat ist.

## Revendications

1. Procédé de fabrication d'un échafaudage poreux tridimensionnel en utilisant une poudre dérivée de tissu animal comprenant :
(a) la réduction en poudre d'un tissu d'origine animale ;
(b) la décellularisation du tissu d'origine animale avant ou après l'avoir réduit en poudre, ou simultanément en le réduisant en poudre ;
(c) le mélange de la poudre de tissu d'origine animale décellularisé avec un porogène sans les dissoudre dans un solvant organique ou dans un autre solvant quelconque, et l'ajout d'un agent de réticulation au mélange afin de réticuler et d'agglomérer la poudre de tissu d'origine animale décellularisé ;
(d) après l'étape (c), la formation de la poudre réticulée de tissu d'origine animale décellularisé en un échafaudage tridimensionnel : et
(e) la formation d'un échafaudage poreux tridimensionnel par le traitement de l'échafaudage tridimensionnel par un procédé de lixiviation des particules qui utilise en tant que porogène au moins une particule choisie dans le groupe constitué d'une particule de sel tel que le chlorure de sodium, une particule de sucre organique, une particule de dextrane, une particule de sucrose, une particule de glace et une particule de paraffine de sorte que le porogène est éliminé de l'échafaudage tridimensionnel.

2. Procédé selon la revendication 1, dans lequel la poudre de tissu d'origine animale décellularisé est transformée en un échafaudage poreux tridimensionnel en mélangeant la poudre de tissu d'origine animale décellularisé et les particules de porogène utilisées dans le procédé de lixiviation des particules, en ajoutant l'agent de réticulation au mélange, en les versant dans un moule, et en les moulant ensuite sous pression pour construire un échafaudage poreux tridimensionnel ayant des formes et des dimensions variées en fonction de la finalité de son usage et du site d'application pour son traitement.

3. Procédé selon l'une quelconque des revendications 1 ou 2, comprenant en outre une nouvelle réticulation de l'échafaudage tridimensionnel obtenu à l'étape (d) par au moins un élément choisi dans le groupe constitué par les UV, l'EDC, le NBS, le procédé déhydrothermique et le glutaraldéhyde.

4. Procédé selon l'une quelconque des revendications 1 ou 2, comprenant en outre l'ajout d'au moins un facteur de croissance à la poudre de tissu d'origine animale et la lyophilisation de ceux-ci.

5. Procédé selon l'une quelconque des revendications 1 ou 2, comprenant en outre l'inoculation de chondrocytes sur ou dans l'échafaudage poreux tridimensionnel formé par le procédé de lixiviation des particules, puis une nouvelle culture des chondrocytes sur ou dans l'échafaudage poreux tridimensionnel et l'obtention d'un tissu de cartilage par ingénierie tissulaire.

6. Procédé selon l'une quelconque des revendications 1 ou 2, dans lequel le tissu d'origine animale peut être du cartilage, ou une membrane amniotique provenant de porcs, de bovins, de moutons, de chevaux, de chiens ou de chats.

7. Procédé selon l'une quelconque des revendications 1 ou 2, dans lequel l'étape de la réduction en poudre du tissu d'origine animale comprend l'isolation du cartilage à partir d'un tissu de cartilage d'origine animale, la pulvérisation du cartilage par un pulvérisateur, et la réduction du cartilage pulvérisé en poudre par un broyeur à froid.

8. Procédé selon l'une quelconque des revendications 1 ou 2, dans lequel la décellularisation est réalisée par une décellularisation physique, une décellularisation chimique, ou par une combinaison de la décellularisation physique et de la décellularisation chimique.

9. Procédé selon la revendication 8, dans lequel la décellularisation physique comprend un gel-dégel, un traitement par ultrasons, ou une agitation physique, et la décellularisation chimique est réalisée en traitant la poudre de tissu d'origine animale avec une solution hypotonique, un détergent anionique, un détergent non ionique, un détergent cationique, de la DNase, de la RNase ou de la trypsine.

10. Procédé selon la revendication 9, dans lequel, dans la décellularisation chimique, la solution hypotonique est une solution de Tris HCL (pH 8,0), le détergent anionique est du dodécylsulfate de sodium (SDS), du désoxycholate de sodium, ou du Triton X 200, le détergent non ionique est du Triton X 100, et le détergent cationique est du CHAPS, de la sulfobétaïne-10 (SB-10), de la sulfobétaïne-16 (SB-16), ou du phosphate de tri-n-butyle.
